# EUROPEAN PATENT APPLICATION

(11) **EP 2 248 887 A1**
(43) Date of publication of application: **10.11.2010**
(21) Application number: 08877353.6
(22) Date of filing: 17.10.2008
(51) Int. Cl.: C12N 5/071, C12N 5/077, A61K 35/50, A61P 3/10, A61P 9/00, A61P 7/00, A61P 25/00, A61P 25/16, A61P 25/28

(54) **A METHOD FOR CONSTRUCTING HUMAN PLACENTAL MESENCHYMAL CELLS LIBRARY WHICH IS SUITABLE FOR CLINICAL APPLICATION**

(71) Applicant: Affiliated Hospital Of Ningxia Medical University, Yinchuan, Ningxia 750004 (CN)
(72) Inventor: YANG, Yinxue, Yinchuan Ningxia 750004 (CN); WEI, Jun, Yinchuan Ningxia 750004 (CN); LI, Yukui, Yinchuan Ningxia 750004 (CN); WANG, Libin, Yinchuan Ningxia 750004 (CN); LIU, Ting, Yinchuan Ningxia 750004 (CN); MA, Xiaona, Yinchuan Ningxia 750004 (CN); ZHANG, Guangyi, Yinchuan Ningxia 750004 (CN)
(74) Representative: O'Neill, Michelle
(86) International application number: PCT/CN2008/001756
(87) International publication number: WO 2010/043076

(57) **Abstract**

The present invention relates to a method for processing human placental cell sample, a human placental cell sample obtained according to said method for processing human placental cell sample, a human placental cell bank, a method for banking human placental cells, a method for searching human placental cell sample in said human placental cell bank according to the present invention, a method for preparing human cord blood serum, use of human placental cells obtained by said method for processing human placental cell sample or human placental cell bank established by said method for banking human placental cells in treating human dysfunction and diseases due to cell injury or cell malfunction, as well as a method for treating human dysfunction and diseases due to cell injury or cell malfunction.

## Description

### Field of the Invention

The present invention relates to the field of medicine technology. Particularly, the present invention relates to a comprehensive method for clinic compliant- expanding human placental mesenchymal stromal cells and establishing and managing a cell bank consisted of said cells, which includes a method for protecting placenta sample, a method for expanding placental mesenchymal stromal cells, a method for preparing human autologous cord blood serum required for implementing these methods, as well as a method for managing and searching the digital registry of said cell bank, and for applying said cells to therapies of human diseases..

### Background Art

Human placental amniotic and chorionic mesenchymal stromal cells contain undifferentiated stem cells from which more identical stem cells can be generated in vitro through cell proliferation, or functional cells of multiple different cell lineages can be generated in vitro through cell differentiation. These two properties of placental stem cells prove to be of great significance to cell transplant therapy due to the large number of committed differentiated functional cells required in the therapy. In addition, placental mesenchymal stromal cells are obtained from the term placenta detached from the mother and infant at the time of birth, therefore the tissue sample is easily procured without any injuries to the mother and infant and does not cause complicated ethical conflict. Furthermore, placental cells have an important immunomodulatory property in the mechanism of protecting a fetus from the influence of allogenic maternal immune system during the fetal development, making them ideal for allogeneic cell transplantation. All together, these features of placental cells verify the high potential for their clinical application in cell therapy-based regenerative medicine. For the general discussion of human placental cells, their potential for clinical applications and laboratory protocols for placental cell processing, please refer to, for examples, Parolini O et al, "Concise Review: Isolation and characterization of cells from human term placenta: Outcome of the First International Workshop on Placenta Derived Stem Cells", STEM CELLS 26, pp300-311, 2008; Ilancheran S et al, "Stem cells derived from human fetal membranes display multi-lineage differentiation potential. Biol Reprod 77, pp577-588, 2007; Portmann-Lanz CB et al, "Placental mesenchymal stem cells as potential autologous graft for pre- and perinatal neuroregeneration", Am J Obstet Gynecol 194, pp664-673, 2006; Yen B et al, "Isolation of multipotent cells from human term placenta", Stem Cells 23, pp3-9, 2005.

In view of the great potential of human stem cells, including human placental stem cells, for clinical applications, the general public is more and more interested in banking stem cells privately and publicly. As a result, several family and public banks of cord blood and cord blood stem cells have been established in many countries. However, so far, human placental amniotic and chorionic mesenchymal stromal cells are mainly preserved for research purpose, and the procedures of processing such cells have not met with the standards for clinical application yet.

In one aspect, most commonly used procedures for expanding human placental amniotic and chorionic mesenchymal stromal cells adopt fetal bovine serum as the main component of nutrient solution. Expanding cells by fetal bovine serum presents potential risks in the following two aspects: 1) introducing virus of animal origin to the cultured cells, and 2) challenging the cultured cells with animal protein antigen. For general discussion on this aspect, please refer to, for example, Mannello F. and Tonti G. "Concise Review: No Breakthroughs for Human Mesenchymal and Embryonic Stem Cell Culture", Stem Cells 25, 1603-1609, 2007.

In another aspect, some previous studies tried to use human cord blood serum as the replacement for animal serum to culture human bone marrow cells and cord blood stem cells (US Patent 7,060,494; US Patent Application 20050059152), but so far there has been no report about culturing human placental mesenchymal stromal cells by utilizing human cord blood serum. Meanwhile, this method also has potential risk of introducing cross contamination with pathogen from different individuals to the cultured cells because the serum from a given blood donor is used for culturing cells of different persons, and some pathogens carried by such human serum may go beyond the scope of current pathogen testing or fail to exceed the sensitivity of current testing techniques. This risk may become crucial as new clinical standards and pathogen testing techniques develop. Therefore the risk must be taken into consideration during cell banking process because most of the cells preserved in the cell bank will be used in several years to tens of years.

In the specification of Chinese patent application CN1407088A (Application Number: 01131190.8), a method was disclosed for releasing hematopoietic stem cells from placenta tissue and banking said hematopoietic stem cells, which comprises steps of separating a cell cluster including placental mesenchymal stromal cells from placenta tissue and cryopreserving said whole cell cluster. There are two disadvantages in that method. Firstly, cell cluster obtained by using that method are all monocytes comprising multiple types of cells including lymphocytes, macrophages and lipocytes and are unsuitable for clinical application. Secondly, cells obtained through that method are directly cryopreserved without culture in vitro, of which the cell survival rate remains to be proven.

Therefore, there exists a practical need for developing a method for banking human placental mesenchymal stromal cells that is suitable for clinical application. The objective of the present invention is to provide an implementation method for satisfying this need.

### Description of the Invention

To facilitate the understanding of this invention, a number of terms are defined below. The terms defined herein have meanings as commonly understood by a person of ordinary skill in the areas relevant to the present invention.

Unless otherwise specified, as used herein the term "term placenta" refers to postpartum placenta of clinically normal pregnancy from either natural delivery or delivery by caesarean section.

Unless otherwise specified, as used herein the term "placental amniotic mesenchymal stromal cells" refers to cells of mesenchymal stromal cells morphology that are released from placental amniotic membrance by digestion with collagenase or any other enzyme or combination of enzymes with similar function.

Unless otherwise specified, as used herein the term "placental chorionic mesenchymal stromal cells" refers to cells of mesenchymal stromal cells morphology that are released from placental chorionic plate by digestion with collagenase or any other enzyme or combination enzymes with similar function.

Unless otherwise specified, as used herein the term "human cord blood serum" refers to serum prepared from a mixture of human umbilical cord blood from different cord blood donors.

Unless otherwise specified, as used herein the term "autologous cord blood serum" refers to serum obtained from cord blood of a given placental cell donor, and the serum herein used is from the same donor who donates placental cells cultured with said serum.

Unless otherwise specified, as used herein the term "DMEM (Dulbecco's modified Eagle's medium)" refers to the basic culture medium well established in the art containing various amino acids and glucose, which can be classified into high glucose form (glucose with a concentration generally of no more than 4500g/L) and low glucose form (glucose with a concentration generally of no more than 1000g/L). A typical example of formulation of DMEM for cell culture is as follows:

| | | | | | |
|---|---|---|---|---|---|
| DMEM for cell culture | | | | | |

| **Component (mg/L)** | **MD200** | **MD201** | **MD202** | **MD203** | **MD204** |
|---|---|---|---|---|---|
| calcium chloride | 200 | 200 | 200 | 200 | 200 |
| ferric nitrate•9H₂O | 0.1 | 0.1 | 0.1 | 0.1 | 0.1 |
| potassium chloride | 400 | 400 | 400 | 400 | 400 |
| anhydrous magnesium sulfate | 97.67 | 97.67 | 97.67 | 97.67 | 97.67 |
| sodium chloride | 6400 | 6400 | 6400 | 6400 | 4400 |
| anhydrous sodium dihydrogen phosphate | 108.7 | 108.7 | 108.7 | 108.7 | 108.7 |
| L-Arginine hydrochloride | 84 | 84 | 84 | 84 | 84 |
| L-Cystine hydrochloride | 63 | 63 | 63 | 63 | 63 |
| L-Glutamine | 584 | 584 | 584 | 584 | 584 |
| Glycine | 30 | 30 | 30 | 30 | 30 |
| L-Histidine hydrochloride | 42 | 42 | 42 | 42 | 42 |
| L-Isoleucine | 105 | 105 | 105 | 105 | 105 |
| L-Leucine | 105 | 105 | 105 | 105 | 105 |
| L-Lysine hydrochloride | 146 | 146 | 146 | 146 | 146 |
| L-Methionine | 30 | 30 | 30 | 30 | 30 |
| L-Phenylalanine | 66 | 66 | 66 | 66 | 66 |
| L-Serine | 42 | 42 | 42 | 42 | 42 |
| L- Threonine | 95 | 95 | 95 | 95 | 95 |
| L- Tryptophane | 16 | 16 | 16 | 16 | 16 |
| L- Tyrosine | 72 | 72 | 72 | 72 | 72 |
| L- Valine | 94 | 94 | 94 | 94 | 94 |
| D- Glucose | 1000 | 4500 | 4500 | 4500 | 4500 |
| phenol red | 15 | 15 | 15 | - | 15 |
| sodium pyruvate | 110 | - | 110 | - | - |
| HEPES | - | - | - | - | 5958 |
| D-calcium pantothenate | 4 | 4 | 4 | 4 | 4 |
| choline chloride | 4 | 4 | 4 | 4 | 4 |
| folic acid | 4 | 4 | 4 | 4 | 4 |
| i-inositol | 7.2 | 7.2 | 7.2 | 7.2 | 7.2 |
| nicotinamide | 4 | 4 | 4 | 4 | 4 |
| pyridoxal hydrochloride | 4 | 4 | 4 | 4 | 4 |
| lactochrome | 0.4 | 0.4 | 0.4 | 0.4 | 0.4 |
| thiamine hydrochloride | 4 | 4 | 4 | 4 | 4 |
| | | | | | |
| pH (without sodium bicarbonate) | 6.3±0.3 | 6.3±0.3 | 6.3±0.3 | 6.3±0.3 | 5.7±0.3 |
| pH (with sodium bicarbonate) | 7.8±0.3 | 7.8±0.3 | 7.8±0.3 | 7.8±0.3 | 7.0±0.3 |
| osmotic pressure(without sodium bicarbonate) | 250±5% | 260±5% | 260±5% | 260±5% | 220±5% |
| osmotic pressure(with sodium bicarbonate) | 316±5% | 335±5% | 335±5% | 335±5% | 300±5% |

Unless otherwise specified, as used herein the term "HLA-typing" refers to any method that can be used to determine HLA type of major histocompatibility (MHC) of a human cell.

Unless otherwise specified, as used herein the term "cell bank" refers to a storage facility of living cells where cells are safely kept for a long term, and where cells from each donor and their information can be individually registered, managed and identified.

Unless otherwise specified, as used herein the term "cGMP" refers to internationally accepted GMP (i.e. Current Good Manufacture Practice, CGMP), which is an industrial code publically used in the world and implemented currently in countries such as the United States, the European countries, Japan and the like.

It is an object of the present invention to provide a clinic compliant method for separating and expanding human placental mesenchymal stromal cells so as to establish a human placental mesenchymal stromal cell bank for clinical application. It is also an object of the present invention to provide a method for growing human placental mesenchymal stromal cells using human cord blood serum. It is still an object of the present invention to provide a method for incubating human placental mesenchymal stromal cells by autologous cord blood serum. It is still further an object of the present invention to provide a method for preparing autologous cord blood serum, synchronizing time for autologous serum preparation with time for placental mesenchymal stromal cell isolation so that the autologous cord blood serum can be used for expanding autologous placental mesenchymal stromal cells. It is yet further an object of the present invention to provide a method for registering and searching human placental cell sample in said human placental cell bank according to the present invention.

In view of the objects of the invention described above, the technical solutions provided in the present invention are as follows:
In one aspect, the present invention provides a method for processing human placental cell sample, and said method comprises:
   a. collecting human term placenta tissue, and protecting the tissue in DMEM containing 0.5% to 5%, preferably 1% of human cord blood serum;
   b. isolating human placental mersenchymal stromal cells from the placenta tissue obtained in step a;
   c. expanding said placental mersenchymal stromal cells obtained in step b in a culture medium that is free of any component of animal origin, and preferably, said culture medium is a DMEM-based culture medium, which further includes: 1) 5%-30%, preferably 10%-20% of human cord blood serum; and 2) 1% of penicillin/streptomycin mixture;
   d. determining antigen type (HLA-typing) of major histocompatibility (MHC) of the placental cells from each cell donor;
   e. bar-coding various HLA-typed cells according to step d and integrating HLA-typing information to registry information data for each cell donor;
   f. protecting said placental mersenchymal stromal cells in cryopreservating solution and storing said cells in liquid nitrogen for a long term, and preferably, said cryopreservating solution consists of 50% human cord blood serum or autologous cord blood serum, 40% DMEM and 10% dimethyl sulphoxide (DMSO).

Preferably, human cord blood serum used in said method is autologous cord blood serum, i.e., said autologous cord blood serum donor is the same one that donates placenta tissue.

Preferably, in said method for processing human placental mersenchymal stromal cell sample according to the present invention:
the process of determining antigen type in step d is implemented using a testing kit, and preferably but not exclusively, said testing kit is a PCR-based testing kit; the bar codes of various HLA-typed cells in step e are generated through an automatic digital bar-coding system, and preferably but not exclusively, said digital bar-coding system is Brady bar coding system TSL2200 (Brady, the United States).

In another aspect, the present invention provides a human placental mersenchymal stromal cell bank, wherein the cell from each donor in said human placental mersenchymal stromal cell bank is obtained through the method of processing human placental cell sample described above.

In a further aspect, the present invention provides a method for banking human placental mersenchymal stromal cells, which comprises:
1) processing human placental mersenchymal stromal cell sample of each person using the method for processing human placental mersenchymal stromal cell sample described above;
2) establishing a searchable record of cell information, said record of cell information is a program for managing bar-coding information and registry information of banked cells in a computer-based program, and said program allows the banked content to be searched by both donor identification (ID) and HLA type, wherein said searchable record of cell information includes:
   a. searching entries: (1) donor identification (ID); (2) HLA type;
   b. donor information: (1) donor's name, address, and phone number of donor's parents, (2) donor's birth date and gender, (3) delivery hospital; and
   c. banking information: (1)name of the person who certifies the cells for banking, (2)number of cells in each storage vial, number of vials stored, and location where each vial is stored, including building name, room number, liquid nitrogen tank number, rack number, cryopreservating box number, and position in the box.

In yet a further aspect, the present invention provides a method for searching human placental mersenchymal stromal cell sample in said human placental mersenchymal stromal cell bank according to the present invention, said method comprising:
1). Setting a registry information record for each sample, of which the content includes:
   a. searching entries: (1) donor identification (ID); (2) HLA type;
   b. donor information: (1) donor's name, address, and phone number of donor's parents, (2) donor's birth date and gender, (3) delivery hospital; and
   c. banking information: (1)name of the person who certifies the cells for banking, (2)number of cells in each storage vial, number of vials stored, and location
      where each vial is stored, including building name, room number, liquid nitrogen tank number, rack number, cryopreservating box number, and position in the box;
2). Determining whether to make the searching information available to the public subject to the requirements of donor's parents; and
3). Adopting a searching engine that is capable of searching the bank content by each and/or all of the searching entries, and preferably but not exclusively, said searching engine is Tiger business management software (HD Tiger, China).

In still a further aspect, the present invention provides a method for preparing autologous cord blood serum, and preferably, said autologous cord blood serum is used as a component of medium for expanding placental mersenchymal stromal cells, wherein said method comprises steps as follows:
a. inserting the needle of a clinic syringe into the umbilical vein at the time of birth and taking the cord blood from the vein into the syringe;
b. transferring the blood to one or more 50 ml centrifuge tubes that are free of anticoagulants;
c. allowing the blood to clot at 37°C for 30 to 60 minutes;
d. cooling the clotted blood at 0 to 5°C for 15 to 45 minutes;
e. having the blood centrifuged under a centrifugal force of 1000g for 10 minutes; and f. transferring said serum to a collecting tube and incubating the serum at 50 to 56°C for 30 minutes.

Preferably, said method synchronizes the time for preparing serum with time for isolating placental mersenchymal stromal cells such that the serum can be used for expanding the placental cells from the same donor.

In yet a further aspect, a method for banking cells is developed in the present invention, wherein information of the banked cells, including HLA type of cells, is managed so that cells from each and all of cell donors can be searched by a bar code generated and a computer-based management program.

In still a further aspect, the present invention provides a use of human placental cells obtained by the method of processing human placental cell sample described above or human placental cell bank established by the method of banking human placental cells described above in treating human dysfunction and diseases caused by cell injury or cell malfunction, and preferably, said human dysfunction and disease due to cell injury or cell malfunction is selected from the group consisting of Type I diabetes, neural injury, myocardial injury, Alzheimer's disease and Parkinson's disease.

In yet a further aspect, the present invention provides a method for treating human dysfunction and diseases caused by cell injury or cell malfunction, said method comprising: using the human placental cells obtained by the method of processing human placental cell sample described above or the human placental cell bank established by the method of banking human placental cells described above, and preferably, said human dysfunction and disease caused by cell injury or cell malfunction is selected from the group consisting of Type I diabetes, neural injury, myocardial injury, Alzheimer's disease and Parkinson's Disease.

According to one preferred embodiment of the present invention, the present invention provides a method for banking human placental cells which is suitable for clinical application, and said method is implemented under the Current Good Manufacture Practice (cGMP) and comprises steps as follows:
a. collecting a human term placenta tissue under aseptic conditions at the time of birth, and protecting the human term placenta tissue in a DMEM with 0.5%-5%, preferably 1% of human cord blood serum added;
b. isolating placental mesenchymal stromal cells from the placenta tissue obtained in step a by a currently available and extensively used method which is well known to those skilled in the art, preferably by a digestion method of collagenase and dispase;
c. expanding said placental mesenchymal stromal cells in a clinical cell culture system that is free of any component of animal origin;
d. determining antigen type (HLA-typing) of major histocompatibility (MHC) of the placental cells from each cell donor;
e. bar-coding the HLA-typed cells and integrating HLA-typing information to registry data for each cell donor;
f. preserving said placental cell in cryopreservating solution and storing said cells in liquid nitrogen for a long term;
g. inputting and saving bar code information and registry information of banked cells into a computer-based program, and said program allows the bank content to be searched by both donor identification (ID) and HLA-type.

According to the method described above, wherein, said clinical cell culture system comprises DMEM, 5% to 30%, preferably, 10% to 20% of human cord blood serum, and 1% of penicillin/streptomycin solution.

According to the method described above, wherein said placental cells are expanded in a clinical cell culture system, said system in vitro includes DMEM, 5% to 30%, preferably 10% to 20% of autologous cord blood serum and 1% of penicillin/streptomycin solution, and said serum is obtained from cord blood of the cell donor, therefore, said serum and said placental cells are from the same donor.

According to the method described above, wherein, HLA type of said placental cells is obtained by a testing kit, and said testing kit is commercially available and well known to those skilled in the art, and preferably but not exclusively, is a PCR-based testing kit.

According to the method described above, wherein, for each cell donor, bar code is generated for each HLA-typed cell through an automatic digital bar-coding system, and preferably but not exclusively, said digital bar-coding system is Brady bar coding system TSL2200 (Brady, the United States).

According to one preferred embodiment of the present invention, the present invention provides a banking registry program used for said method of the present invention, wherein, said banking registry includes:
a. searching entries: (1) donor ID; (2)HLA type;
b. donor information: (1)donor's name, address, and phone number of donor's parents, (2)donor's birth date and gender, (3)delivery hospital;
c. banking information: (1)name of the person who certifies the cells for banking, (2)number of cells in each storage vial, number of vials stored, and location where each vial is stored, including building name, room number, liquid nitrogen tank number, rack number, cryopreservating box number, and position in the box.
d. informed content from donor's parents to certify whether or not to make the searching information available to the public;
e. searching engine that is capable of searching the bank content by each and/or all of the searching entries, and preferably but not exclusively, said searching engine is Tiger business management software (HD Tiger, China).

Accoding to the method described above, wherein, said cryopreservating solution comprises 50% human cord blood serum or autologous cord blood serum, 40% DMEM and 10% dimethyl sulphoxide (DMSO).

According to another preferred embodiment of the present invention, the present invention provides autologous cord blood serum prepared through the steps as follows:
a. inserting the needle of a clinic syringe into the umbilical vein and taking the cord blood from the vein into the syringe;
b. transferring the blood to a 50 ml centrifuge tube that is free of anticoagulants;
c. allowing the blood to clot at 37°C for 30 to 60 minutes under cGMP environment;
d. cooling the clotted blood at 0 to 5°C for 15 to 45 minutes;
e. having the blood centrifuged under a centrifugal force of 1000g for 10 minutes
f. transferring serum to a collecting tube and incubating the serum at 50 to 56°C for 30 minutes.

According to a preferred embodiment of the present invention, the present invention provides a method for banking and maintaining human placental mesenchymal stromal cells. In this method, human placental mesenchymal stromal cells are expanded by human cord blood serum or autologous cord blood serum, and said cell are processed and banked under clinic-applicable conditions, then the information of said banked cells is managed in a searchable data base. Whether the clinic-relevant information of each and all of cell donors in the data base can be searched or not is determined by the will of cell's donor.

As described herein, in one embodiment, the present invention provides a procedure for banking human mesenchymal stromal cells. The procedure includes the followings: first, the procedure provides a method for collecting human placenta tissue from a delivery room. The placenta tissue is collected under aseptic conditions and protected in a solution containing 1% of human cord blood serum; second, the procedure provides steps for isolation and in vitro expansion of placental mesenchymal stromal cells. In such steps, placental mesenchymal stromal cells are expanded in a medium comprising human cord blood serum and being free of any component of animal origin; third, the procedure provides a method for HLA-typing of cells to be banked. The method employs DNA-based HLA-typing and is independent from antigen expression; forth, the procedure provides a method for bar-coding and banking cells. This bar-coding and computer input is performed by an automatic bar-coding system and matched with data management and searching based on the computer; fifth, the procedure also provides a format for data entries, management and searching of the cell bank. This format includes information of cells, cell donor and cell processing method.

In one embodiment, the present invention provides a method for expanding placental mesenchymal stromal cells by autologous cord blood serum. In this method, during in vitro expansion, said placental mesenchymal stromal cells are exposed to no biological components different from those they are exposed to in the placenta; hence the possibility of contamination from cell culture components with biological pathogens is eliminated.

In another embodiment, the present invention provides a method for preparing autologous cord blood serum for growing placental cells. The method synchronizes the time for autologous serum preparation with that for placental cell isolation so that the preparation of the serum is completed when the cells from the same placenta are ready to be cultured.

In another embodiment, the present invention provides a method for HLA-typing of placental cells. In this method, HLA types of the cells to be banked are determined, preferably but not exclusively, by a DNA -based HLA-typing method. The DNA-based HLA-typing method provides an advantage that the HLA typing is independent from cell differentiation, and thereby suitable for HLA-typing of all kinds of placental cells.

In one specific aspect, the present invention provides a method for bar-coding banked cells.

In this method, a unique bar code is generated, preferably but not exclusively, by Brady bar coding system for cells from each cell donor, and integrated into a computer-based database.

In another aspect, the present invention also provides a format that integrates bar code, HLA types, donor information, cell characteristics, and also differentiation for whether to enter a searchable data base for the public, so that the cell bank can accommodate different banking ways for families and general public simultaneously.

Typical steps and sequence of the method according to the present invention will be described in details below according to one particular example of the present invention:

### 1. Isolation of placental amniotic and chorionic mesenchymal stromal cells

At the time of birth, a part of placenta tissue is dissected from the term placenta under aseptic conditions, and the dissected tissue is protected in a centrifuge tube containing DMEM with addition of 1% of antibiotics and 1% of human cord blood serum. The placenta tissue is transferred to a processing lab within 30 minutes and washed three times with PBS containing 1% of penicillin/streptomycin solution.

Chorionic plate is dissected from the placenta tissue and washed with the PBS three times as described above. To isolate placental amniotic and chorionic mesenchymal stromal cells, chorionic plate is subject to digestion with a combination of dispase and collagenase for 30 minutes to 2 hours. The dispase may be used at 2 to 4 units per ml, and collagenase may be used at 200 to 400 units per ml and the digestion temperature is at 37°C. After digestion, the tissue debris in digestion content is allowed to sit down for 30 to 60 seconds and cells in suspension are collected by centrifugation for in vitro expansion.

### 2. In vitro expansion of placental mesenchymal stromal cells

The human placental mesenchymal stromal cells isolated from placenta tissue, including placental amniotic and chorionic mesenchymal stromal cells, are washed in PBS with addition of antibiotics and serum, and plated in cell culture flask containing DMEM supplemented with 10% to 20% of human cord blood serum or 10% to 20% of autologous cord blood serum, and 1% of antibiotic (complete medium). The cells are cultured in 5% CO₂ air of 37°C. After one week, the culture medium is replaced with complete fresh medium and the culture is continued for one more week. Cells are subcultured at the end of the second week and in every 3 to 4 days thereafter.

### 3. Preparation of autologous cord blood serum

Cord blood of each placental cell donor is collected and processed separately. The cord blood is collected at the time of birth using a 50 ml clinic syringe with a needle. Insert the needle into the umbilical vein of a placenta and the cord blood is taken from the umbilical vein into the syringe. The blood is then transferred to a 50 ml centrifuge tube which is free of anticoagulants. And then the blood is allowed to clot at 37°C for 30 to 60 minutes under cGMP environment. After the clotting process, the clotted blood is cooled at 0 to 5°C for 15 to 45 minutes and then is centrifuged at 1000g for 10 minutes. The serum is transferred to a collecting tube and inactivated at 50 to 56°C for 30 minutes.

### 4. HLA-typing of placental cells

DNA sample of placental cells from each cell donor is prepared using a DNA isolation kit that is commercially available and familiar to those skilled in the art. Such DNA sample from each cell donor is used for PCR-based HLA typing using a commercially available HLA typing kit and familiar to those skilled in the art.

### 5. Digital bar-coding and registration for banking

In vitro expanded placental mesenchymal stromal cells are cryopreserved in liquid nitrogen at 1.2 million cells per vial. Cell strain in each vial is bar-coded with an automatic bar code generator, and the bar code together with information of the cell strain and its donor is input to a computer-based management system through a registry program. Said registry program includes:
a. searching entries:(1)donor ID, (2)HLA types;
b. donor information: (1)name, address, and phone number of donor's parents, (2)donor's birth date and gender, (3)delivery hospital;
c. banking information: (1)name of the person who certifies the cells for banking, (2)number of cells in each storage vial, number of vials stored, and location where each vial is stored, including building name, room number, liquid nitrogen tank number, rack number, cryopreservating box number, and position in the box;
d. request of donor's parents for determining whether to make the searching information available to the public;
e. searching engine that can search the bank content using each and all of the searching entries.

Compared to the prior art, the originality of the present invention primarily reflects in several aspects as follows:
First, the present invention provides a method for expanding human placental mesenchymal stromal cells with human cord blood serum. The placental mesenchymal stromal cells are directly developed from the inner cell of an early embryo without committed differentiation to any cell lineage, and their development process and cellular characteristics are different from any type of adult cells, including adult bone marrow stem cells and cord blood stem cells concerned in prior study. In the body, the placental mesenchymal stromal cells are directly nourished by cord blood; hence, we hypothesize that in vitro cord blood serum can simulate the nutritive environment in which in vivo placental stem cells maintain their undifferentiated state, so, expanding placental mesenchymal stromal cells with cord blood serum can not only maintain cell growth, but also retain the original properties of stem cells. This hypothesis has been proven true by the study leading to the present invention.
Either cell type or culture mechanism in the prior attempts of expanding human bone marrow cells and cord blood stem cells is different from the disclosure of the present invention.

Second, the present invention provides a method for expanding human placental mesenchymal stromal cells with autologous cord blood serum and a method for preparation of the autologous cord blood serum synchronized with isolation of the placental mesenchymal stromal cells. The combination of both the two methods allows the cells expanded according to the method to be safely used in clinical application without any pathological risks. There has been no report about expanding human placental mesenchymal stromal cells with autologous blood serum before.

Third, the present invention provides a clinic-applicable method for banking human placental mesenchymal stromal cells. One part of this method includes isolating and expanding placental cells in a system that is free of any component of animal origin to obtain human placental mesenchymal stromal cells that are suitable for clinical application. In the specification of Chinese patent application CN1407088A (Application Number: 01131190.8), a method was disclosed of obtaining hematopoietic stem cells from placenta tissue and banking said hematopoietic stem cells. As described above, the method comprises steps of separating a cell cluster including placental mesenchymal stromal cells from placenta tissue and cryopreserving said whole cell cluster. The main point of the method is: 1. The placenta tissue (not singular cell) was washed and protected in mixed solution of cord blood plasma (not serum) and DMEM and stored at 4°C for no more than 24 hours; 2. All the monocytes were isolated from the palcenta tissue protected in plasma and DMEM, and directly freezed and preserved (for details, please refer to Example 1 and Example 2 on page 11-12 and "Particular Embodiments" portion of the text of the application on page 8-9). All of the parts associated with cell culture in the method were related to identification by sampling cells and the culture method was not related to serum or plasma, and in addition, the expanded cells were only used for identification and not for cryopreservation. There are two disadvantages of that method. First, the cell cluster obtained through that method are all types of monocytes comprising several types of cells including lymphocytes, macrophages and lipocytes, and are unsuitable for clinic application. Second, the cells obtained according to that method are directly cryopreserved without in vitro culture, of which the survival rate remains to be proven. Different from the method described in said patent application, the cell bank provided in the present invention is established from placental mesenchymal stromal cells which are expanded in vitro before cryopreservation, and a method for in vitro expanding human placental cells with human cord blood serum before cryopreservation. As a result, more pure clinic-applicable placental mesenchymal stromal cells with a higher survival rate after cryopreservation can be provided by the present invention. Therefore, the method of the present invention is totally different from the method disclosed in the earlier application document in terms of operation, and is obviously better than the prior relative methods on technical effect.

Other aspects of the present invention have been described in details in the description of the invention and will be illustrated in the embodiments below. In view of the foregoing description, it will become apparent to those skilled in the art that equivalent modifications thereof may be made without departing from the theory and technical scope of this invention and are within the protection scope claimed by the present invention.

### Brief Description of the Drawings

The following figures are incorporated herein to further demonstrate certain aspects of the present invention. The invention may be better understood by reference to these figures in combination with the detailed description presented herein, wherein:
Figure 1: This figure shows the morphology of human placental mesenchymal stomal cells growing in DMEM containing 10% of fetal bovine serum.
Figure 2: This figure shows the morphology of human placental mesenchymal stomal cells growing in DMEM containing 10% of human non-antologous cord blood serum.
Figure 3: This figure shows the morphology of human placental mesenchymal stomal cells growing in DMEM containing 10% of human antologous cord blood serum.

### Best Modes for Carrying Out the Invention

Herein the present invention will be further illustrated by making reference to particular examples. However, these examples are only limited to describing the present invention, and are not used to limit the scope of the present invention. Experimental methods without indicating specific experimental conditions are generally in conformity with conventional conditions or conditions suggested by manufacturers.

### Example 1: Isolation of human placental amniotic mesenchymal stromal cells and human placental chorionic mesenchymal stromal cells

Fresh human placenta tissue of about 20 g was dissected from a human term placenta at the time of birth under aseptic conditions. The tissue was stored in a 50 ml centrifuge tube containing 20 ml of DMEM (Invitrogen, product code: 11885084) having 1% of human cord blood serum and 1% of penicillin/ streptomycin solution (Invitrogen, product code: 15140122). The tissue in the protective solution was transferred to a cGMP laboratory within 30 minutes, and was washed three times in PBS (Invitrogen, product code: 14040133) containing 1% penicillin/ streptomycin solution before processing.

To isolate placental amniotic and chorionic mesenchymal stromal cells, chorionic plate was dissected from the placenta tissue with an aseptic surgery scissor, washed three times in the PBS, cut into pieces of about 1 mm³ in size, and then digested with a combination of collagenase IV (Invitrogen, 17104019) at 270 units per ml and Dispase II (Roche, product type 04942078001) at 2.4 units per ml for 1 hour at 37°C. After digestion, tissue debris in the digestion content was allowed to sit down for 30 seconds, and then the middle layer of the cell suspension was collected. The collected cell suspension was diluted with equal volume of PBS and centrifuged at 700g for 10 minutes, and then the supernatant was poured away. The cells in debris were washed 2 times in PBS containing 1% human cord blood serum, and then once in DMEM containing 1% human cord blood serum.

0.5 to 1 million placental mesenchymal stromal cells could be obtained from fresh placenta tissue of 100 g according to the method described in this example.

### Example 2: Preparation of autologous cord blood serum and human cord blood serum for placental cell growth

Cord blood of each placental cell donor was collected and processed separately according to the following method. The cord blood was collected at the time of birth using a 50 ml clinic syringe with a 16G needle. The needle was inserted into the umbilical vein of the placenta and the cord blood was taken from the umbilical vein into the syringe. The blood was then transferred to a 50 ml centrifuge tube that is free of anticoagulants. 30 to 40 ml of cord blood was collected in each tube. And the blood was transferred to a cGMP laboratory within 30 minutes.

### 2.1 Preparation of autologous cord blood serum

According to different donors, the collected cord blood in centrifuge tubes that are free of anticoagulants was respectively clotted for 45 minutes at 37°C, cooled in ice water for 30 minutes and then centrifuged at 1000g for 10 minutes at room temperature. The serum on top of the tube content was transferred to a new tube and centrifuged one more time under the same condition. The serum in the supernatant was transferred to a new tube and incubated at 56°C for 30 minutes. Such serum could be preserved at 4°C for one week or at -20°C for 6 months. 30 to 40 ml of autologous cord blood serum could be obtained from 100ml cord blood according to this method.

### 2.2 Preparation of non-autologous cord blood serum (also known as human cord blood serum herein)

The collected cord blood from different donors in centrifuge tubes that are free of anticoagulants was mixed and transferred separately to new centrifuge tubes that are free of anticoagulants. Then the blood serum was allowed to be clotted at room temperature for 16 hours. Then the clotted blood was cooled at 4°C for 2 hours and centrifuged at 1000g for 10 minutes at room temperature. The serum on top of the tube content was transferred to a new tube and centrifuged one more time under the same condition. Then the serum in the supernatant was transferred to a new tube and incubated at 56°C for 30 minutes. Such serum could be preserved at 4°C for one week or at -20°C for 6 months.

30 to 40 ml of human cord blood serum could be obtained from each cord blood of 100ml according to this method.

### Example 3: In vitro expansion of human placental mesenchymal stromal cells using human cord blood serum

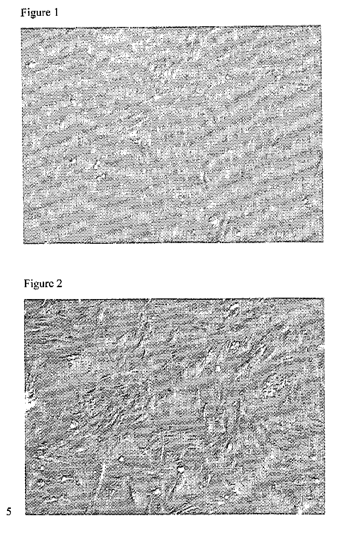

The human placental amniotic and chorionic mesenchymal stromal cells obtained according to Example 1 were dispersed in complete DMEM at a concentration of 1×10⁶ (one million) cells per ml medium and plated in a 25 cm² tissue culture flask at a volume of 7.5 ml per flask. The components in said complete DMEM included: 89% of DMEM, 10% of human cord blood serum and 1% penicillin/streptomycin solution. The cells in flask were cultured in 37°C 5% CO₂ air. After one week, the culture medium was replaced with fresh complete DMEM medium and continued to be cultured for one more week. Cells were subcultured at the end of the second week and in every 3 to 4 days thereafter using fresh complete medium. The method for each subculture was: the culture medium in culture flask was removed with an aseptic sucker on a clean bench, and the cell layer grown on the surface of the flask bottom was washed with PBS once. After the PBS was removed, 1ml of 1% trypase solution (Invitrogen, product code: 25300) was added to cover the whole cell layer. The cells were incubated at 37°C for 1 minute and the addition of 5 ml of complete DMEM was followed. Cells in such culture medium were mixed into a uniform suspension of singular cells, and equally separated into three new tissue culture flask of 25 cm² and complete DMEM was added to each flask to a volume of 7.5 ml. The cells and medium were mixed and then the flasks were put into a 37°C incubator containing 5% CO₂ air. One week later, placental amniotic and chorionic mesenchymal stromal cells and amniotic epithelial cells in part in the human placental cells expanded according to this method could complete the first division cycle with doubled quantity, and other types of cells, including lymphocytes, macrophages and lipocytes would have been eliminated naturally to obtain a comparatively pure cell cluster. From the second week, the cells entered normal cell development cycle, and morphology of the cells was observed every 3 to 4 days and analysis of growth activity was performed by cell number counting under a microscope. Results of cell morphology and analysis of growth activity from this example were shown in figure 2 and table 1 attached below.

### Example 4: In vitro expansion of human placental mesenchymal stromal cells using autologous cord blood serum

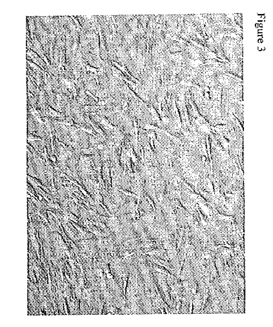

Human placental amniotic and chorionic mersenchymal stromal cells obtained according to the method in Example 1 were expanded in vitro. The procedure implemented, reagents used and method for analyzing cell growth were the same as those described in Example 3 with the exception that human cord blood serum was replaced with autologous cord blood serum. Results of cell morphology and analysis of growth activity from this example were shown in figure 3 and table 1 attached below.

### Example 5: In vitro expansion of human placental mersenchymal stromal cells using fetal bovine serum

This example was used as a control experiment for Example 3 and 4. Human placental amniotic and chorionic mersenchymal stromal cells obtained according to Example 1 were expanded in vitro. The procedure implemented, reagents used and method for analyzing cell growth were the same as those described in Example 3 with the exception that human cord blood serum was replaced with fetal bovine serum (Invitrogen, product code: 10099141). Results of cell morphology and analysis of growth activity from this example were shown in figure 1 and table 1 attached below.

**Table 1: comparison of cell growth activity of human placental mersenchymal stromal cells in different serum**

| **serum used** | **time for cell culture** | **cell division cycle** |
|---|---|---|
| fetal bovine serum | generation 2 to 6 | 24 to 30 hours |
| autologous human cord blood serum | generation 2 to 6 | 24 to 30 hours |
| non-autologous human cord blood serum | generation 2 to 4 | 24 to 36 hours |

From Figures 1 to 3 and Table 1 above, it is illustrated that, compared to expansion method usually using fetal bovine serum in the prior art, human placental mersenchymal stromal cells expanded using human cord blood serum and autologous cord blood serum can achieve the same result as, or even a better result than cells expanded using fetal bovin serum in terms of both cell morphology and growth activity, which verifies that the method for expanding human placental mersenchymal stromal cells provided in the present invention can be used to replace the previously used method and also satisfy the requirements of clinic applications.

## Claims

1. A method for processing human placental cell sample, **characterized in that** said method comprises steps as follows:
a. collecting human term placenta tissue, and protecting the tissue in DMEM containing 0.5% to 5%, preferably 1% of human cord blood serum;
b. isolating human placental amniotic and chorionic mersenchymal stromal cells from the placenta tissue obtained in step a;
c. in vitro expanding said human placental cells in a cell culture system that is free of any component of animal origin, and preferably said culture system is a DMEM-based medium, which further includes:
1). 5%-30%, preferably 10%-20% of human cord blood serum; and
2). 1% of penicillin/streptomycin solution;
d. determining antigen type (HLA-typing) of major histocompatibility (MHC) of the placental cells from each cell donor;
e. bar-coding the various HLA-typed cells obtained in step d and integrating HLA type information to registry information data for each cell donor;
f. preserving said placental mersenchymal stromal cells in cryopreservating solution and storing said cells in liquid nitrogen, and preferably, said cryopreservating solution is consisted of 50% human cord blood serum or autologous cord blood serum, 40% DMEM and 10% dimethyl sulphoxide (DMSO).

2. A method according to claim 1, **characterized in that** the human cord blood serum used in said method is autologous cord blood serum which is obtained from autologous cord blood of the given placental mersenchymal stromal cell donor, therefore, said autologous cord blood serum and the placental mersenchymal stromal cells cultured with said serum are from the same donor.

3. A method according to claim 1, **characterized in that**:
the antigen type in step d is determined using a testing kit, and preferably but not exclusively, said testing kit is a PCR-based testing kit; and/or
bar codes of the various HLA-typed cells in step e are generated using an automatic digital bar-coding system, and preferably but not exclusively, said digital bar-coding system is Brady bar coding system TSL2200 (Brady, the United States).

4. Human placental mersenchymal stromal cells obtained using the method according to any one of claims 1 to 3.

5. A human placental mersenchymal stromal cell bank, **characterized in that** placental mersenchymal stromal cells from each donor in said human placental mersenchymal stromal cell bank are obtained using the method according to any one of claims 1 to 3.

6. A method for banking human placental mersenchymal stromal cells, **characterized in that** said method comprises:
1) processing human placental mersenchymal stromal cell sample of each person using the method according to any one of claims 1 to 3;
2) establishing searchable record of cell information, and said record of cell information is a program for registering and managing bar-coding information and information of banked cells in a computer-based program which allows the bank content to be searched by both donor identification (ID) and HLA type.

7. A method for banking human placental mersenchymal stromal cells according to claim 6, **characterized in that** said searchable record of cell information includes:
a. searching entries: (1) donor identification (ID) and (2) HLA type;
b. donor information: (1) donor's name, address, and phone number of donor's parents, (2) donor's birth date and gender, (3) delivery hospital; and
c. banking information: (1)name of the person who certifies the cells for banking, (2) number of cells in each storage vial, number of vials stored, and location where each vial is stored, including building name, room number, liquid nitrogen tank number, rack number, cryopreservating box number, and position in the box.

8. A method for searching human placental cell sample in said human placental cell bank according to claim 5, said method comprising:
1) setting record of registry information for each sample, of which the content includes:
a. searching entries: (1) donor ID, (2) HLA type;
b. donor information: (1) donor's name, address, and phone number of donor's parents, (2) donor's birth date and gender, (3)delivery hospital; and
c. banking information: (1)name of the person who certifies the cells for banking, (2) number of cells in each storage vial, number of vials stored, and location where each vial is stored, including building name, room number, liquid nitrogen tank number, rack number, cryopreservating box number, and position in the box;
2) requests of donor's parents for determining whether to make searching information available to the public; and
3) searching engine that is capable of searching the bank content using each and/or all of the searching entries respectively, and preferably but not exclusively, said searching engine is Tiger business management software (HD Tiger, China).

9. A method for preparing autologous cord blood serum, and preferably said autologous cord blood serum is used as a component of medium for expanding placental mersenchymal stromal cells, wherein said method comprises steps as follows:
a. inserting the needle of a clinic syringe into the umbilical vein at the time of birth and taking the cord blood from the vein into the syringe;
b. transferring the blood to a 50 ml centrifuge tube that is free of anticoagulants;
c. allowing the blood to clot at 37°C for 30 to 60 minutes;
d. cooling the clotted blood at 0 to 5 °C for 15 to 45 minutes;
e. having the blood centrifuged at 1000g for 10 minutes; and
f. transferring the serum to a collecting tube and incubating the serum at 50 to 56°C for 30 minutes.

10. Use of human placental cells obtained using the method according to any one of claims 1 to 3 or human placental cell bank established using the method according to claim 6 or 7 in treating human dysfunction and diseases due to cell injury or cell malfunction, and preferably, said human dysfunction and disease due to cell injury or cell malfunction is selected from the group consisting of Type I diabetes, neural injury, myocardial injury, Alzheimer's disease and Parkinson's Disease.

11. A method for treating human dysfunction and diseases due to cell injury or cell malfunction, said method comprising: using human placental cells obtained by the method according to any one of claims 1 to 3 or human placental cell bank established by the method according to claims 6 or 7, wherein preferably said human dysfunction and disease due to cell injury or cell malfunction is selected from the group consisting of Type I diabetes, neural injury, myocardial injury, Alzheimer's disease and Parkinson's Disease.
